# EUROPEAN PATENT APPLICATION

(11) **EP 4 763 095 A1**
(43) Date of publication of application: **24.06.2026**
(21) Application number: 24853457.0
(22) Date of filing: 12.07.2024
(51) Int. Cl.: A61B 8/12

(54) **ULTRASONIC DETECTION AUXILIARY SYSTEM FOR CATHETER AND METHOD THEREFOR**

(30) Priority: 17.08.2023 US 202363533210 P
(71) Applicant: Yeh, Chih-Kuang, Hsinchu City Taiwan 30013 (TW)
(72) Inventor: TSAI, Chieh-Yu, Hsinchu City Taiwan 30013 (CN); YEH, Chih-Kuang, Hsinchu City Taiwan 30013 (CN)
(74) Representative: Kuhnen & Wacker Patent- und Rechtsanwaltsbüro PartG mbB
(86) International application number: PCT/CN2024/105084
(87) International publication number: WO 2025/036045

(57) **Abstract**

Provided are an ultrasonic detection auxiliary system for a catheter and a method therefor. The ultrasonic detection auxiliary system for a catheter is suitable for being introduced into a cardiovascular system to alleviate obstruction in the cardiovascular system or a surrounding tissue thereof. The ultrasonic detection auxiliary system for a catheter includes: an ultrasonic balloon catheter with ultrasonic probes configured to continuously emit an ultrasonic wave and obtain a calcified vascular reflection signal; and a control host, further including: a signal receiving unit electrically connected to the ultrasonic probes and configured to receive a calcification reflection analog signal; a digital-to-analog conversion circuit configured to receive the calcified vascular reflection signal and generate a calcification characteristic signal; a duplex circuit coupled to the signal receiving unit and the digital-to-analog conversion circuit; a processing unit electrically connected to the digital-to-analog conversion circuit and configured to analyze and calculate the calcification characteristic signal to generate a control signal to enhance or reduce output energy of the ultrasonic wave; and an amplification circuit electrically connected to the processing unit and configured to amplify a resonance signal according to the control signal, to cause the ultrasonic probes to emit the ultrasonic wave.

## Description

### TECHNICAL FIELD

The present application relates to an ultrasonic detection auxiliary system for a catheter and a method therefor, and in particular, to an ultrasonic detection auxiliary system for a catheter for vascular therapy and a method therefor, which can receive a vascular calcification fragmentation degree through ultrasonic probes to adjust an intensity and a direction of ultrasonic waves.

### BACKGROUND

Cardiovascular diseases rank first among the top ten causes of death worldwide for a long term. With the aging population and gradual westernization of diet, the prevalence and mortality rates of cardiovascular diseases have been increasing year by year, and there is a trend of younger patients with cardiovascular diseases. Coronary artery disease (CAD) and peripheral artery disease (PAD) are the most common cardiovascular diseases. The two diseases are mainly due to the deposition and hardening of fat or other substances in the arteries, which form atheromatous plaques or calcifications, resulting in arteriosclerosis and obstruction to normal blood flow. It is conceivable that insufficient or complete absence of blood flow in the coronary arteries may cause heart disease or even lead to heart failure and sudden death. In addition, peripheral arterial disease may cause pain during walking or poor wound healing in limbs. In severe cases, the peripheral arterial disease may lead to critical limb ischemia (CLI), resulting in amputation.

Vascular calcification may cause the vascular wall to harden and lose its due elasticity, resulting in poor contraction and relaxation of the blood vessels. Moreover, excessive calcium deposition on the blood vessel wall can be called vascular calcification. Calcification may lead to vascular stenosis, resulting in poor blood flow. Generally, there are many manners of treating vascular stenosis, such as laser or rotational atherectomy. The most common and cheapest method is percutaneous transluminal angioplasty (PTA), commonly known as balloon catheter dilation, which inflates the balloon by using water pressure, to restore a narrowed blood vessel. However, when there are too severe symptoms of sclerosis, it often leads to the failure to inflate the balloon smoothly and rupture, resulting in the risk of surgery.

### SUMMARY

An objective of the present application is to provide an ultrasonic detection auxiliary system for a catheter and a method therefor, to induce a cavitation through an ultrasonic probe capable of emitting an ultrasonic wave, and obtain a vascular calcification fragmentation status through ultrasonic reflection or scattering, so as to adjust an intensity and a direction of ultrasonic waves in time, and effectively alleviate arterial vascular obstruction or refractory vascular calcification.

To achieve the foregoing objective, the present application provides an ultrasonic detection auxiliary system for a catheter, suitable for being introduced into a cardiovascular system to alleviate obstruction in the cardiovascular system or a surrounding tissue thereof, including: an ultrasonic balloon catheter with at least two ultrasonic probes configured to continuously emit an ultrasonic wave and obtain a calcified vascular reflection signal; and a control host, further including: a signal receiving unit electrically connected to the at least two ultrasonic probes and configured to receive a calcification reflection analog signal; a digital-to-analog conversion circuit configured to receive the calcified vascular reflection signal and generate a calcification characteristic signal; a duplex circuit coupled to the signal receiving unit and the digital-to-analog conversion circuit; a processing unit electrically connected to the digital-to-analog conversion circuit and configured to analyze and calculate the calcification characteristic signal to generate a correlation, a sample entropy, a Shannon entropy, an approximate entropy, and an envelope SNR, generate a calcification data value according to the correlation, and generate a control signal according to the calcification data value, the sample entropy, the Shannon entropy, the approximate entropy, and the envelope SNR to enhance or reduce output energy of the ultrasonic wave; and an amplification circuit electrically connected to the processing unit and configured to amplify a resonance signal according to the control signal, to cause the ultrasonic probes to emit the ultrasonic wave.

In an embodiment of the present application, the ultrasonic balloon catheter includes a multi-lumen catheter, a flexible hose with at least one opening, and a balloon made of a polymer material and provided with an expansion part for expansion and contraction and necks arranged at both ends of the expansion part respectively, where the balloon is fixedly arranged outside the multi-lumen catheter by using the necks, and the expansion part and the necks form an expansion space outside the multi-lumen catheter, to inject a fluid composition with microbubbles through each of the openings.

In an embodiment of the present application, the at least two ultrasonic probes are arranged inside the expansion part of the balloon, sleeved on the multi-lumen catheter, and configured to emit an ultrasonic wave to induce an inertial cavitation generated by the microbubbles in the fluid composition located in the expansion space.

In an embodiment of the present application, the multi-lumen catheter includes an inner tube and an outer tube, which are arranged as a concentric circle structure, the inner tube forms a guide wire lumen, a guide wire is arranged in the guide wire lumen, the ultrasonic balloon catheter slides between blood vessels by using the guide wire, the expansion part of the balloon is placed at a vascular calcified part, and the outer tube has at least one lumen, which includes a microbubble lumen for injecting the fluid composition, and a power lumen for allowing a wire providing power required by the ultrasonic probes to pass through.

In an embodiment of the present application, in the multi-lumen catheter, the inner tube has an outer diameter of 0.5 mm to 1 mm and an inner diameter of 0.3 mm to 0.8 mm, and the outer tube has an outer diameter of 2 mm and an inner diameter of 0.5 mm to 1 mm.

In an embodiment of the present application, the ultrasonic balloon catheter further includes a catheter assembly, one end of the catheter assembly is connected to the multi-lumen catheter, the other end thereof is provided with a guide wire channel and a fluid composition channel, the guide wire channel allows the guide wire to pass through to enter the guide wire lumen, and the fluid composition channel allows the fluid composition to be injected into the microbubble lumen.

In an embodiment of the present application, the at least two ultrasonic probes may include a transmitting probe for emitting the ultrasonic wave and a receiving probe for obtaining the calcified vascular reflection signal, and the transmitting probe and the receiving probe are arranged on the ultrasonic balloon catheter at an interval.

In an embodiment of the present application, the at least two ultrasonic probes are each a tubular ultrasonic probe, and the ultrasonic probe has an inner diameter of 0.5 mm to 1 mm, an outer diameter of 1.5 mm to 2.5 mm, a height of 2 mm to 6 mm, and resonant frequencies of 100 kHz to 2 MHz and 2 MHz to 10 MHz.

In an embodiment of the present application, grounds on outer surfaces of the at least two ultrasonic probes are segmented by cutting out gaps of 0.01 mm to 0.2 mm at an interval between 90° and 180°.

The present application further includes an ultrasonic detection auxiliary method for a catheter, suitable for being introduced into a cardiovascular system to alleviate obstruction in the cardiovascular system or a surrounding tissue thereof, including the following steps: emitting an ultrasonic wave by an ultrasonic probe in an ultrasonic balloon catheter, and obtaining a calcified vascular reflection signal by another ultrasonic probe; receiving a calcification reflection analog signal by a signal receiving unit of a control host; converting the calcified vascular reflection signal into a calcification characteristic signal by a digital-to-analog conversion circuit; analyzing and calculating, by a processing unit, the calcification characteristic signal to generate a correlation, and performing cross correlation comparison between the correlation and a sample in a database to determine a calcification type and generate a calcification data value; separately calculating, by the processing unit, a sample entropy, a Shannon entropy, an approximate entropy, and an envelope SNR according to the calcification characteristic signal, and performing comparison with a sample entropy threshold, a Shannon entropy threshold, an approximate entropy threshold, and an envelope SNR threshold in the database respectively, so as to determine a calcification fragmentation level; determining, by the processing unit, whether vascular calcification fragmentation is completed according to the calcification data value and the calcification fragmentation level; and generating a control signal to stop emitting the ultrasonic wave, when the vascular calcification fragmentation has been completed, or generating the control signal, when the vascular calcification fragmentation is not completed, adjusting an intensity of the ultrasonic wave by an amplification circuit, and after a preset time, obtaining the calcified vascular reflection signal again by the signal receiving unit and performing analysis and calculation.

The ultrasonic detection auxiliary system for a catheter and the method therefor according to the present application feature at least the following: The cavitation is induced by emitting ultrasonic waves from the transmitting probe among a plurality of ultrasonic probes, and an ultrasonic reflection signal is received by the receiving probe to learn about the vascular calcification fragmentation status, so as to adjust an intensity and a direction of ultrasonic waves in time, and effectively alleviate arterial vascular obstruction or refractory vascular calcification.

### BRIEF DESCRIPTION OF THE DRAWINGS

FIG. 1 is a schematic block diagram of an ultrasonic detection auxiliary system for a catheter according to the present application;
FIG. 2 is a schematic structural diagram of an ultrasonic detection auxiliary system for a catheter according to the present application;
FIG. 3 is a schematic diagram of an ultrasonic balloon catheter of an ultrasonic detection auxiliary system for a catheter according to the present application;
FIG. 4A to FIG. 4D are schematic diagrams of a multi-lumen catheter of an ultrasonic detection auxiliary system for a catheter according to the present application;
FIG. 5 is a schematic diagram of an ultrasonic probe of an ultrasonic detection auxiliary system for a catheter according to the present application;
FIG. 6 is a schematic diagram of an ultrasonic balloon catheter according to Embodiment 1 of the present application;
FIG. 7 is an operation flowchart of an ultrasonic detection auxiliary system for a catheter according to Embodiment 1 of the present application;
FIG. 8 is a schematic diagram of an ultrasonic balloon catheter according to Embodiment 2 of the present application;
FIG. 9 is an operation flowchart of an ultrasonic detection auxiliary system for a catheter according to Embodiment 2 of the present application;
FIG. 10 is a step flowchart of an ultrasonic detection auxiliary method for a catheter according to the present application; and
FIG. 11 is a schematic explanatory diagram of a cavitation.

In the figures:
11: ultrasonic balloon catheter; 111: ultrasonic probe; 1111: transmitting probe; 1112: receiving probe; 1113: gap; 1114: fire; 1115: ground; 112: multi-lumen catheter; 1121: opening; 113: balloon; 1131: expansion part; 1132: neck; 12: control host; 121: signal receiving unit; 122: digital-to-analog conversion circuit; 123: duplex circuit; 124: processing unit; 125: amplification circuit; 13: catheter assembly; 131: guide wire channel; 132: fluid composition channel; 2: multi-lumen catheter; 21: inner tube; 211: guide wire lumen; 22: outer tube; 221: microbubble lumen; 222: power lumen; 2221: wire; S101 to S107B: step flow.

### DESCRIPTION OF EMBODIMENTS

To make the foregoing features and advantages of the present application more obvious and easier to understand, the following embodiments are given, and with reference to the accompanying drawings, in addition to these detailed descriptions, the present application can also be widely applied to other embodiments. Any easy substitution, modification and equivalent changes of the embodiments fall within the scope of the present application, and are subject to the claims.

In the description of the specification, many specific details are provided for readers to have a more complete understanding of the present application. However, the present application may still be implemented while some or all specific details are omitted. In addition, well-known steps or elements are not described in details to avoid unnecessarily limiting the present application. The same or similar elements in the drawings are denoted by the same or similar symbols. It should be specially noted that the drawings are only for illustrative purposes and do not denote actual sizes or quantities of elements. Some details may not be fully drawn, to simplify the drawings. Detailed explanations are provided below.

The following embodiments are illustrated with reference to the accompanying drawings, to illustrate specific embodiments based on which the present application can be implemented. Directional terms mentioned in the present application, such as "upper", "lower", "front", "rear", "left", "right", "inside", "outside", and "side", are only directions with reference to the accompanying drawings. Therefore, the directional terms used are used to illustrate and understand the present application, not to limit the present application. In addition, in the specification, unless explicitly described to the contrary, the term "including" is to be understood as meaning including the element, but not excluding any other elements.

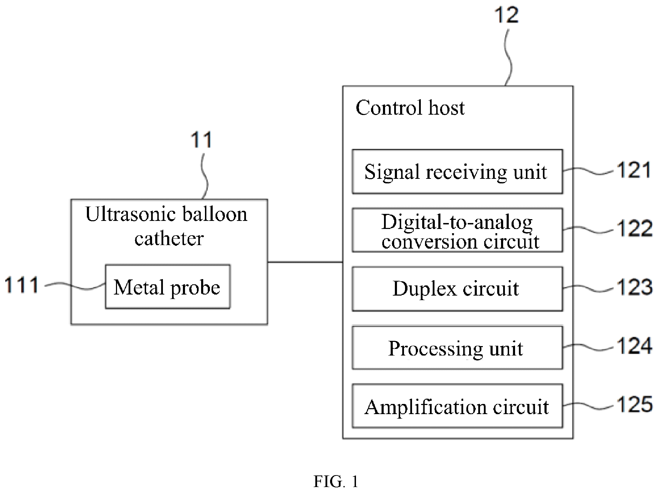

FIG. 1 is a schematic block diagram of an ultrasonic detection auxiliary system for a catheter according to the present application. The ultrasonic detection auxiliary system for a catheter shown in FIG. 1 is suitable for being introduced into a cardiovascular system to alleviate obstruction in the cardiovascular system or a surrounding tissue thereof. The ultrasonic detection auxiliary system for a catheter includes: an ultrasonic balloon catheter 11 with at least two ultrasonic probes 111 configured to continuously emit an ultrasonic wave and obtain a calcified vascular reflection signal; and a control host 12, further including: a signal receiving unit 121 electrically connected to the at least two ultrasonic probes 111 and configured to receive a calcification reflection analog signal; a digital-to-analog conversion circuit 122 configured to receive the calcified vascular reflection signal and generate a calcification characteristic signal; a duplex circuit 123 coupled to the signal receiving unit 121 and the digital-to-analog conversion circuit 122; a processing unit 124 electrically connected to the digital-to-analog conversion circuit 122 and configured to analyze and calculate the calcification characteristic signal to generate a correlation, a sample entropy, a Shannon entropy, an approximate entropy, and an envelope SNR, generate a calcification data value according to the correlation, and generate a control signal according to the calcification data value, the sample entropy, the Shannon entropy, the approximate entropy, and the envelope SNR to enhance or reduce output energy of the ultrasonic wave; and an amplification circuit 125 electrically connected to the processing unit 124 and configured to amplify a resonance signal according to the control signal, to cause the ultrasonic probes 111 to emit the ultrasonic wave.

The cavitation, also referred to as the cavitation phenomenon, refers to the effect generated by microbubbles, which is induced when ultrasonic waves with certain energy and audio frequency are applied to the microbubbles.

The inertial cavitation refers to the phenomenon in which microbubbles are extremely compressed and expanded due to strong acoustic energy of ultrasonic waves, resulting in pulse wave and liquid jet caused by the final collapse. The forces can enhance drug absorption of a target site.

In this embodiment, the ultrasonic balloon catheter 11 includes a multi-lumen catheter, a flexible hose with at least one opening, and a balloon made of a polymer material and provided with an expansion part for expansion and contraction and necks arranged at both ends of the expansion part respectively, where the balloon is fixedly arranged outside the multi-lumen catheter by using the necks, and the expansion part and the necks form an expansion space outside the multi-lumen catheter, to inject a fluid composition with microbubbles through each of the openings.

In this embodiment, the at least two ultrasonic probes 111 are arranged inside the expansion part of the balloon, sleeved on the multi-lumen catheter, and configured to emit an ultrasonic wave to induce an inertial cavitation generated by the microbubbles in the fluid composition located in the expansion space.

In this embodiment, the multi-lumen catheter includes an inner tube and an outer tube, which are arranged as a concentric circle structure, the inner tube forms a guide wire lumen, a guide wire is arranged in the guide wire lumen, the ultrasonic balloon catheter 11 slides between blood vessels by using the guide wire, the expansion part of the balloon is placed at a vascular calcified part, and the outer tube has at least one lumen, which includes a microbubble lumen for injecting the fluid composition, and a power lumen for allowing a wire providing power required by the ultrasonic probes 111 to pass through.

When the multi-lumen catheter passes through a vascular network of the cardiovascular system, the multi-lumen catheter is fitted with a guide wire path and slides along the guide wire.

The multi-lumen catheter is circular, and the balloon can be smoothly guided by the guide wire to an affected part for treatment.

The circular structure of the multi-lumen catheter enables each component to reach the affected part through a blood vessel without being damaged and operate smoothly.

In this embodiment, in the multi-lumen catheter, the inner tube has an outer diameter of 0.5 mm to 1 mm and an inner diameter of 0.3 mm to 0.8 mm, and the outer tube has an outer diameter of 2 mm and an inner diameter of 0.5 mm to 1 mm.

The outer tube is made of a soft material, and the soft material includes, but is not limited to at least one of a metal, an epoxy resin, an acrylic resin, a polyurethane (PU) resin, a polyimide (PI) resin, rubber, silicone, plastic nylon, or another polymer material.

In this embodiment, the ultrasonic balloon catheter 11 further includes a catheter assembly, one end of the catheter assembly is connected to the multi-lumen catheter, the other end thereof is provided with a guide wire channel and a fluid composition channel, the guide wire channel allows the guide wire to pass through to enter the guide wire lumen, and the fluid composition channel allows the fluid composition to be injected into the microbubble lumen.

The catheter assembly may be, but is not limited to, a Luer taper.

Further, when it is determined that microbubbles are insufficient, the fluid composition with microbubbles is injected from the catheter assembly according to the control signal.

When it is determined that microbubbles are insufficient, prompt information is displayed on the ultrasonic detection auxiliary system for a catheter according to the control signal, to remind a user to inject the fluid composition with microbubbles.

In this embodiment, the at least two ultrasonic probes 111 may include a transmitting probe for emitting the ultrasonic wave and a receiving probe for obtaining the calcified vascular reflection signal, and the transmitting probe and the receiving probe are arranged on the ultrasonic balloon catheter 11 at an interval.

With two ultrasonic probes 111 as an example, the one close to the front end of the balloon is a transmitting probe, and the other close to the rear end of the balloon is a receiving probe.

With three ultrasonic probes 111 as an example, two ultrasonic probes at the front and the rear of the ultrasonic balloon catheter 11 are transmitting probes, and the middle ultrasonic probe is a receiving probe.

With five ultrasonic probes 111 as an example, three ultrasonic probes at the front, the middle, and the rear of the ultrasonic balloon catheter 11 are transmitting probes, and receiving probes are arranged between the transmitting probes at intervals.

The transmitting probes may be connected in parallel, or may be wired independently.

In this embodiment, an end of the ultrasonic balloon catheter 11 is connected to the control host 12, and the receiving probe is connected to the signal receiving unit 121, the duplex circuit 123, and the digital-to-analog conversion circuit 122. The transmitting probe is connected to the amplification circuit 125 and finally connected to the control host 12.

The duplex circuit 123 is implemented by using a coupler or a duplexer.

The wire for powering the ultrasonic probe 111 may be, but is not limited to, an enameled wire, a centrally conductive metal tube, a copper sticker or another connection manner with high conductivity efficiency.

In this embodiment, the at least two ultrasonic probes 111 are each a tubular ultrasonic probe, and the ultrasonic probe 111 has an inner diameter of 0.5 mm to 1 mm, an outer diameter of 1.5 mm to 2.5 mm, a height of 2 mm to 6 mm, and resonant frequencies of 100 kHz to 2 MHz and 2 MHz to 10 MHz.

The at least two ultrasonic probes 111 may be made of, but not limited to, a piezoelectric material.

A surface electrode coating on each of the at least two ultrasonic probes 111 may be, but is not limited to, electroless nickel immersion gold (NiAu), silver (Ag), or another highly conductive material.

The at least two ultrasonic probes 111 are each made of a piezoelectric material with a metal coating plated on its surface.

In this embodiment, grounds on outer surfaces of the at least two ultrasonic probes 111 are segmented by cutting out gaps of 0.01 mm to 0.2 mm at an interval between 90° and 180°.

The segmented electrodes receive calcified vascular reflection signals with different angles through each gap, so as to clearly determine the reflection angles of the calcified vascular reflection signals.

At least one wire is connected to a fire of the ultrasonic probes 111.

At least one wire is connected to an earth electrode of the ultrasonic probes 111.

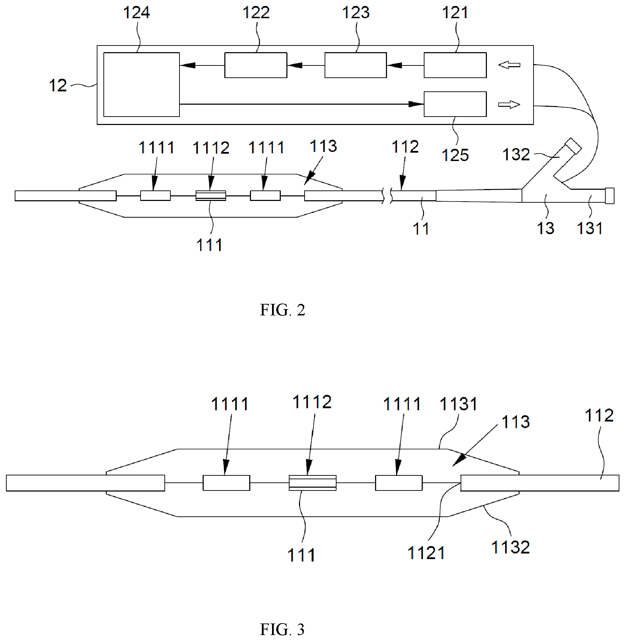

FIG. 2 is a schematic structural diagram of an ultrasonic detection auxiliary system for a catheter according to the present application. The ultrasonic detection auxiliary system for a catheter shown in FIG. 2 is suitable for being introduced into a cardiovascular system to alleviate obstruction in the cardiovascular system or a surrounding tissue thereof. The ultrasonic detection auxiliary system for a catheter includes: an ultrasonic balloon catheter 11 with at least two ultrasonic probes 111 configured to continuously emit an ultrasonic wave and obtain a calcified vascular reflection signal; and a control host 12, further including: a signal receiving unit 121 electrically connected to the at least two ultrasonic probes 111 and configured to receive a calcification reflection analog signal; a digital-to-analog conversion circuit 122 configured to receive the calcified vascular reflection signal and generate a calcification characteristic signal; a duplex circuit 123 coupled to the signal receiving unit 121 and the digital-to-analog conversion circuit 122; a processing unit 124 electrically connected to the digital-to-analog conversion circuit 122 and configured to analyze and calculate the calcification characteristic signal to generate a correlation, a sample entropy, a Shannon entropy, an approximate entropy, and an envelope SNR, generate a calcification data value according to the correlation, and generate a control signal according to the calcification data value, the sample entropy, the Shannon entropy, the approximate entropy, and the envelope SNR to enhance or reduce output energy of the ultrasonic wave; and an amplification circuit 125 electrically connected to the processing unit 124 and configured to amplify a resonance signal according to the control signal, to cause the ultrasonic probes 111 to emit the ultrasonic wave.

In this embodiment, the ultrasonic balloon catheter 11 includes a multi-lumen catheter 112, a flexible hose with at least one opening, and a balloon 113 made of a polymer material and provided with an expansion part for expansion and contraction and necks arranged at both ends of the expansion part respectively. The balloon 113 is fixedly arranged outside the multi-lumen catheter 112 by using the necks, and the expansion part and the necks form an expansion space outside the multi-lumen catheter 112, to inject a fluid composition with microbubbles through each of the openings.

In this embodiment, the at least two ultrasonic probes 111 are arranged inside the expansion part of the balloon 113, sleeved on the multi-lumen catheter 112, and configured to emit an ultrasonic wave to induce an inertial cavitation generated by the microbubbles in the fluid composition located in the expansion space.

In this embodiment, the multi-lumen catheter 112 includes an inner tube and an outer tube, which are arranged as a concentric circle structure, the inner tube forms a guide wire lumen, a guide wire is arranged in the guide wire lumen, the ultrasonic balloon catheter 11 slides between blood vessels by using the guide wire, the expansion part of the balloon 113 is placed at a vascular calcified part, and the outer tube has at least one lumen, which includes a microbubble lumen for injecting the fluid composition, and a power lumen for allowing a wire providing power required by the ultrasonic probes 111 to pass through.

In this embodiment, in the multi-lumen catheter 112, the inner tube has an outer diameter of 0.5 mm to 1 mm and an inner diameter of 0.3 mm to 0.8 mm, and the outer tube has an outer diameter of 2 mm and an inner diameter of 0.5 mm to 1 mm.

In this embodiment, the ultrasonic balloon catheter 11 further includes a catheter assembly 13, one end of the catheter assembly 13 is connected to the multi-lumen catheter 112, the other end thereof is provided with a guide wire channel 131 and a fluid composition channel 132, the guide wire channel 131 allows the guide wire to pass through to enter the guide wire lumen, and the fluid composition channel 132 allows the fluid composition to be injected into the microbubble lumen.

In this embodiment, the at least two ultrasonic probes 111 may include a transmitting probe 1111 for emitting the ultrasonic wave and a receiving probe 1112 for obtaining the calcified vascular reflection signal, and the transmitting probe 1111 and the receiving probe 1112 are arranged on the ultrasonic balloon catheter 11 at an interval.

In this embodiment, with three ultrasonic probes 111 as an example, two ultrasonic probes at the front and the rear of the ultrasonic balloon catheter 11 are transmitting probes 1111, and the middle ultrasonic probe is a receiving probe 1112.

In this embodiment, an end of the ultrasonic balloon catheter 11 is connected to the control host 12, and the receiving probe 1112 is connected to the signal receiving unit 121, the duplex circuit 123, and the digital-to-analog conversion circuit 122. The transmitting probe 1111 is connected to the amplification circuit 125 and finally connected to the control host 12.

In this embodiment, the at least two ultrasonic probes 111 are each a tubular ultrasonic probe, and the ultrasonic probe 111 has an inner diameter of 0.5 mm to 1 mm, an outer diameter of 1.5 mm to 2.5 mm, a height of 2 mm to 6 mm, and resonant frequencies of 100 kHz to 2 MHz and 2 MHz to 10 MHz.

The at least two ultrasonic probes 111 may be made of, but not limited to, a piezoelectric material.

A surface electrode coating on each of the at least two ultrasonic probes 111 may be, but is not limited to, electroless nickel immersion gold (NiAu), silver (Ag), or another highly conductive material.

The at least two ultrasonic probes 111 are each made of a piezoelectric material with a metal coating plated on its surface.

In this embodiment, grounds on outer surfaces of the at least two ultrasonic probes 111 are segmented by cutting out gaps of 0.01 mm to 0.2 mm at an interval between 90° and 180°.

FIG. 3 is a schematic diagram of an ultrasonic balloon catheter 11 of an ultrasonic detection auxiliary system for a catheter according to the present application. Referring to FIG. 3, the ultrasonic balloon catheter 11 includes a multi-lumen catheter 112, a flexible hose with at least one opening, and a balloon 113 made of a polymer material and provided with an expansion part 1131 for expansion and contraction and necks 1132 arranged at both ends of the expansion part 1131 respectively. The balloon 113 is fixedly arranged outside the multi-lumen catheter 112 by using the necks 1132, and the expansion part 1131 and the necks 1132 form an expansion space outside the multi-lumen catheter 112, to inject a fluid composition with microbubbles through each of the openings 1121.

In this embodiment, the at least two ultrasonic probes 111 are arranged inside the expansion part 1131 of the balloon 113, sleeved on the multi-lumen catheter 112, and configured to emit an ultrasonic wave to induce an inertial cavitation generated by the microbubbles in the fluid composition located in the expansion space.

In this embodiment, the multi-lumen catheter 112 includes an inner tube and an outer tube, which are arranged as a concentric circle structure, the inner tube forms a guide wire lumen, a guide wire is arranged in the guide wire lumen, the ultrasonic balloon catheter 11 slides between blood vessels by using the guide wire, the expansion part 1131 of the balloon 113 is placed at a vascular calcified part, and the outer tube has at least one lumen, which includes a microbubble lumen for injecting the fluid composition, and a power lumen for allowing a wire providing power required by the ultrasonic probes 111 to pass through.

In this embodiment, in the multi-lumen catheter 112, the inner tube has an outer diameter of 0.5 mm to 1 mm and an inner diameter of 0.3 mm to 0.8 mm, and the outer tube has an outer diameter of 2 mm and an inner diameter of 0.5 mm to 1 mm.

In this embodiment, the ultrasonic balloon catheter 11 further includes a catheter assembly, one end of the catheter assembly is connected to the multi-lumen catheter 112, the other end thereof is provided with a guide wire channel and a fluid composition channel, the guide wire channel allows the guide wire to pass through to enter the guide wire lumen, and the fluid composition channel allows the fluid composition to be injected into the microbubble lumen.

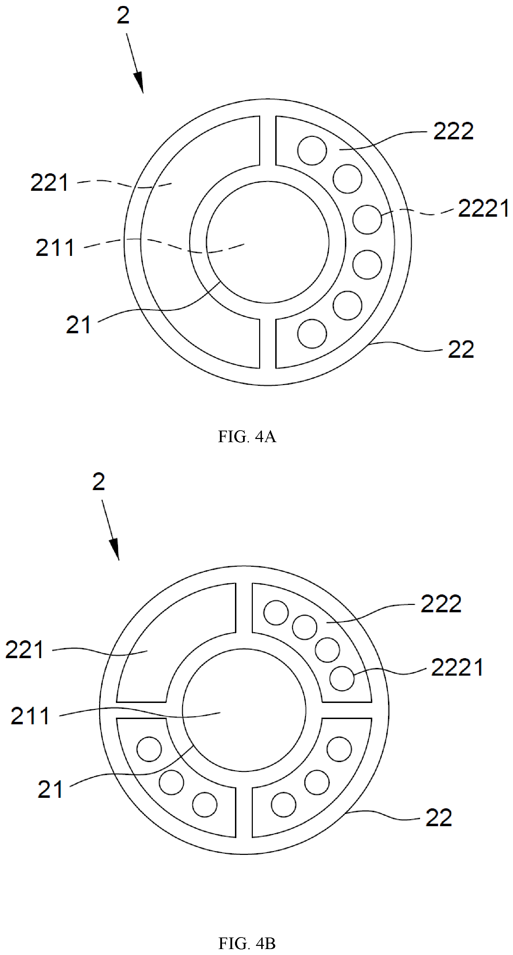

Refer to FIG. 4A to FIG. 4D. FIG. 4A to FIG. 4D are schematic diagrams of a multi-lumen catheter of an ultrasonic detection auxiliary system for a catheter according to the present application.

As shown in FIG. 4A, the multi-lumen catheter 2 includes an inner tube 21 and an outer tube 22, which are arranged as a concentric circle structure. The inner tube 21 forms a guide wire lumen 211, and a guide wire is arranged in the guide wire lumen 211. The outer tube 22 has two lumens, including a microbubble lumen 221 for injecting the fluid composition, and a power lumen 222 for allowing a wire 2221 providing power required by the ultrasonic probes to pass through.

As shown in FIG. 4B, the multi-lumen catheter 2 includes an inner tube 21 and an outer tube 22, which are arranged as a concentric circle structure. The inner tube 21 forms a guide wire lumen 211, and a guide wire is arranged in the guide wire lumen 211. The outer tube 22 has four lumens, including a microbubble lumen 221 for injecting the fluid composition, and three power lumens 222 for allowing a wire 2221 providing power required by the ultrasonic probes to pass through.

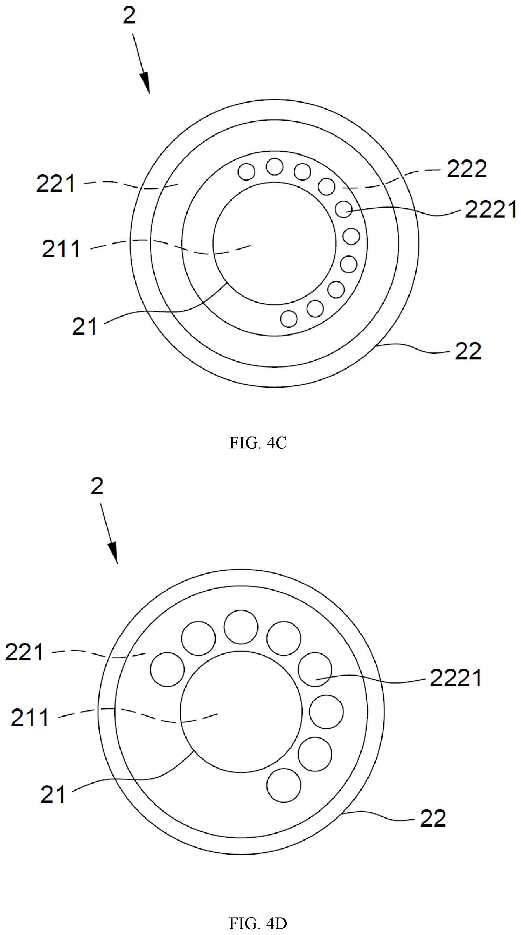

As shown in FIG. 4C, the multi-lumen catheter 2 includes an inner tube 21 and an outer tube 22, which are arranged as a concentric circle structure. The inner tube 21 forms a guide wire lumen 211, and a guide wire is arranged in the guide wire lumen 211. The outer tube 22 has two lumens arranged as a concentric circle structure, including a microbubble lumen 221 for injecting the fluid composition, and a power lumen 222 for allowing a wire 2221 providing power required by the ultrasonic probes to pass through.

As shown in FIG. 4D, the multi-lumen catheter 2 includes an inner tube 21 and an outer tube 22, which are arranged as a concentric circle structure. The inner tube 21 forms a guide wire lumen 211, and a guide wire is arranged in the guide wire lumen 211. The outer tube 22 has one lumen, and the lumen is a microbubble lumen 221 for injecting the fluid composition and for allowing a wire 2221 providing power required by the ultrasonic probes to pass through.

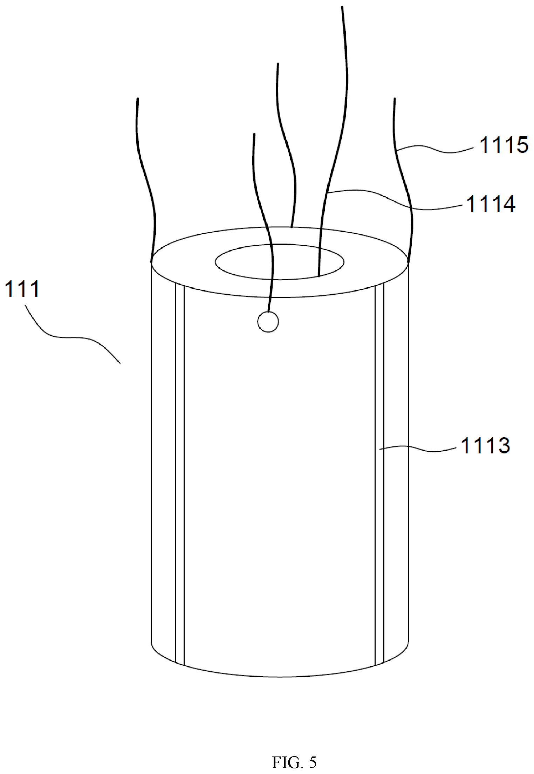

FIG. 5 is a schematic diagram of an ultrasonic probe of an ultrasonic detection auxiliary system for a catheter according to the present application. The ultrasonic probe 111 shown in FIG. 5 is a tubular ultrasonic probe, and the ultrasonic probe 111 has an inner diameter of 0.5 mm to 1 mm, an outer diameter of 1.5 mm to 2.5 mm, a height of 2 mm to 6 mm, and resonant frequencies of 100 kHz to 2 MHz and 2 MHz to 10 MHz.

The at least two ultrasonic probes 111 may be made of, but not limited to, a piezoelectric material.

A surface electrode coating on each of the at least two ultrasonic probes 111 may be, but is not limited to, electroless nickel immersion gold (NiAu), silver (Ag), or another highly conductive material.

The at least two ultrasonic probes 111 are each made of a piezoelectric material with a metal coating plated on its surface.

In this embodiment, grounds on outer surfaces of the at least two ultrasonic probes are segmented by cutting out gaps 1113 of 0.01 mm to 0.2 mm at an interval between 90° and 180°.

A reflection angle of a calcified vascular reflection signal can be clearly determined through each gap 1113.

At least one wire is connected to a fire 1114 of each ultrasonic probe.

At least one wire is connected to a ground 1115 of each ultrasonic probe.

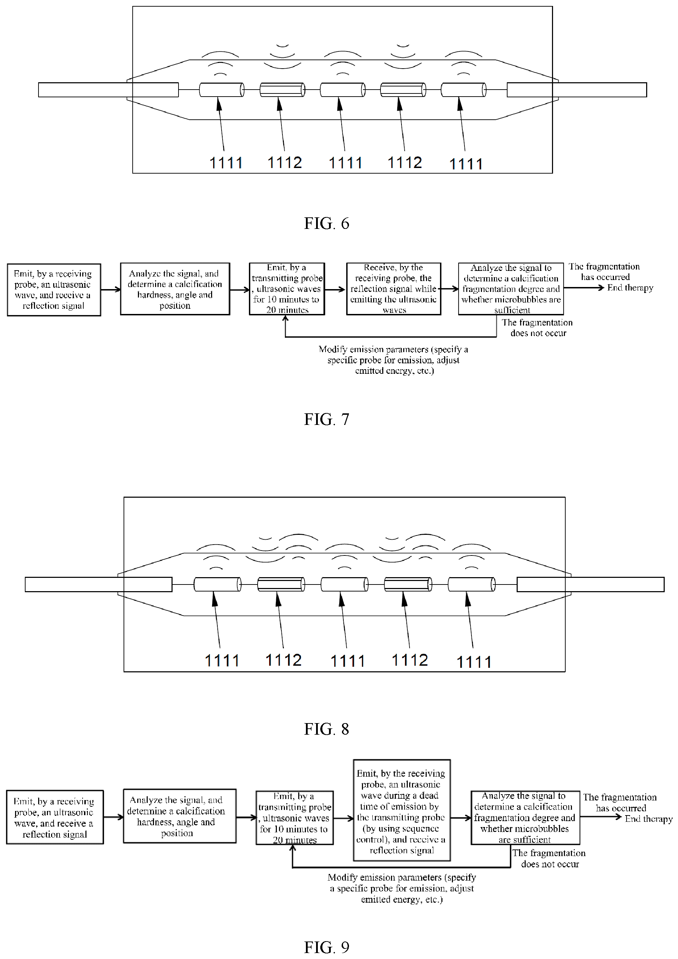

Refer to FIG. 6 and FIG. 7. FIG. 6 is a schematic diagram of an ultrasonic balloon catheter according to Embodiment 1 of the present application. FIG. 7 is an operation flowchart of an ultrasonic detection auxiliary system for a catheter according to Embodiment 1 of the present application. In an ultrasonic detection auxiliary system for a catheter as shown in FIG. 6, with five ultrasonic probes as an example, three ultrasonic probes at the front, the middle, and the rear of the ultrasonic balloon catheter are transmitting probes 1111, and receiving probes 1112 are arranged between the transmitting probes 1111 at intervals.

In this embodiment, ultrasonic waves are transmitted by the transmitting probes 1111, and calcified vascular reflection signals are received by the receiving probes 1112.

A calcification characteristic signal is analyzed and calculated by a processing unit, to generate a correlation, and cross correlation comparison is performed between the correlation and a sample in a database to determine a calcification type and generate a calcification data value.

The calcification data value includes a calcification hardness, a calcification angle, and a calcification position.

The calcification position is a position set relative to the receiving probes 1112.

A sample entropy, a Shannon entropy, an approximate entropy, and an envelope SNR are separately calculated by the processing unit according to the calcification characteristic signal, and comparison is performed with a sample entropy threshold, a Shannon entropy threshold, an approximate entropy threshold, and an envelope SNR threshold in the database respectively, so as to determine a calcification fragmentation level.

Through the processing unit, the transmitting probes 1111 continuously emit ultrasonic waves for 10 minutes to 20 minutes, and the receiving probes 1112 receive a calcification reflection analog signal.

A control signal is generated to stop emitting the ultrasonic waves, when vascular calcification fragmentation has been completed.

Alternatively, when the vascular calcification fragmentation is not completed, the control signal is generated, and an intensity of the ultrasonic waves is adjusted by an amplification circuit.

Refer to FIG. 8 and FIG. 9. FIG. 8 is a schematic diagram of an ultrasonic balloon catheter according to Embodiment 2 of the present application. FIG. 9 is an operation flowchart of an ultrasonic detection auxiliary system for a catheter according to Embodiment 2 of the present application. In an ultrasonic detection auxiliary system for a catheter as shown in FIG. 8, with five ultrasonic probes as an example, three ultrasonic probes at the front, the middle, and the rear of the ultrasonic balloon catheter are transmitting probes 1111, and receiving probes 1112 are arranged between the transmitting probes 1111 at intervals.

The receiving probes 1112 can simultaneously emit ultrasonic waves.

In this embodiment, the transmitting probes 1111 only emit the ultrasonic waves to induce an inertial cavitation generated by microbubbles, and the receiving probes 1112 emit ultrasonic waves and receive a calcified vascular reflection signal.

A calcification characteristic signal is analyzed and calculated by a processing unit, to generate a correlation, and cross correlation comparison is performed between the correlation and a sample in a database to determine a calcification type and generate a calcification data value.

The calcification data value includes a calcification hardness, a calcification angle, and a calcification position.

The calcification position is a position set relative to the receiving probes 1112.

A sample entropy, a Shannon entropy, an approximate entropy, and an envelope SNR are separately calculated by the processing unit according to the calcification characteristic signal, and comparison is performed with a sample entropy threshold, a Shannon entropy threshold, an approximate entropy threshold, and an envelope SNR threshold in the database respectively, so as to determine a calcification fragmentation level.

Through the processing unit, the transmitting probes 1111 continuously emit ultrasonic waves for 10 minutes to 20 minutes, and by using sequence control, after the transmitting probes 1111 emit the ultrasonic waves, the receiving probes 1112 emit ultrasonic waves instead and receive a calcification reflection analog signal.

A control signal is generated to stop emitting the ultrasonic waves, when vascular calcification fragmentation has been completed.

Alternatively, when the vascular calcification fragmentation is not completed, the control signal is generated, and an intensity of the ultrasonic waves is adjusted by an amplification circuit.

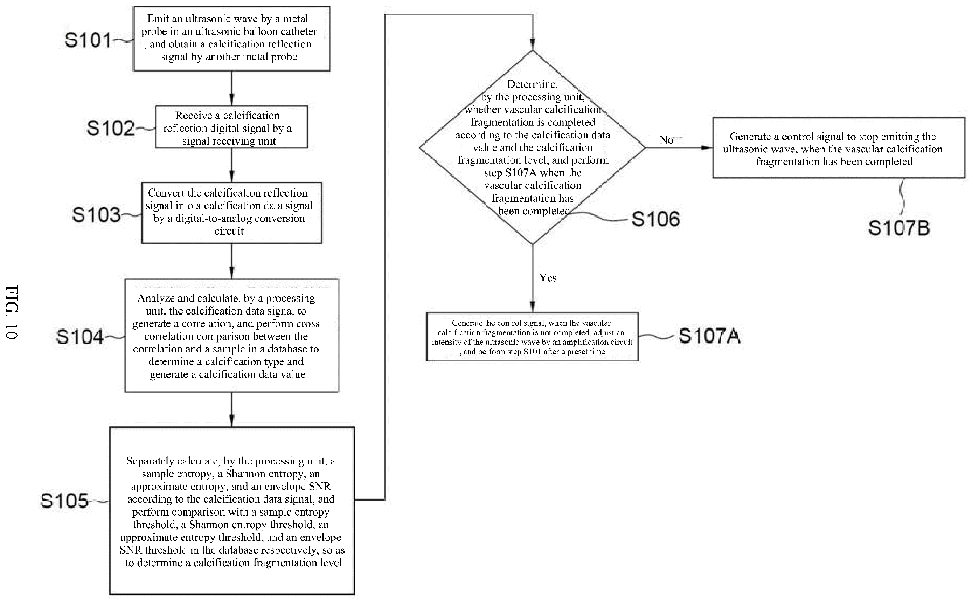

FIG. 10 is a step flowchart of an ultrasonic detection auxiliary method for a catheter according to the present application. The ultrasonic detection auxiliary method for a catheter in FIG. 10 is suitable for being introduced into a cardiovascular system to alleviate obstruction in the cardiovascular system or a surrounding tissue thereof, and includes the following steps:
Step S101: Emit an ultrasonic wave by an ultrasonic probe in an ultrasonic balloon catheter, and obtain a calcified vascular reflection signal by another ultrasonic probe.
Step S102: Receive a calcification reflection analog signal by a signal receiving unit.
Step S103: Convert the calcified vascular reflection signal into a calcification characteristic signal by a digital-to-analog conversion circuit.
Step S104: Analyze and calculate, by a processing unit, the calcification characteristic signal to generate a correlation, and perform cross correlation comparison between the correlation and a sample in a database to determine a calcification type and generate a calcification data value.

The processing unit performs cross correlation comparison between the calcification characteristic signal and a sample signal in the database, classifies the calcification characteristic signal to determine the calcification type, and performs weighted adjustment on the calcification characteristic signal.

Step S105: Separately calculate, by the processing unit, a sample entropy, a Shannon entropy, an approximate entropy, and an envelope SNR according to the calcification characteristic signal, and perform comparison with a sample entropy threshold, a Shannon entropy threshold, an approximate entropy threshold, and an envelope SNR threshold in the database respectively, so as to determine a calcification fragmentation level.

The foregoing four types of entropy of the calcification characteristic signal are calculated, and are compared with the corresponding entropy thresholds.

When two or more types of entropy in four types of entropy are greater than entropy thresholds, the calcification fragmentation level is determined.

The calcification fragmentation level is divided as follows:
1. No fragmentation.
2. Slight fragmentation: for example, there is a single or two cracks.
3. To be fragmented: for example, there are a plurality of cracks.
4. Complete fragmentation: for example, there are a plurality of large cracks, and a vascular lumen is dilated.

Step S106: Determine, by the processing unit, whether vascular calcification fragmentation is completed according to the calcification data value and the calcification fragmentation level, and perform step S107A when the vascular calcification fragmentation has been completed.

Step S107A: Generate a control signal to stop emitting the ultrasonic wave, when the vascular calcification fragmentation has been completed.

Step S107B: Generate the control signal, when the vascular calcification fragmentation is not completed, adjust an intensity of the ultrasonic wave by an amplification circuit, and perform step S101 after a preset time.

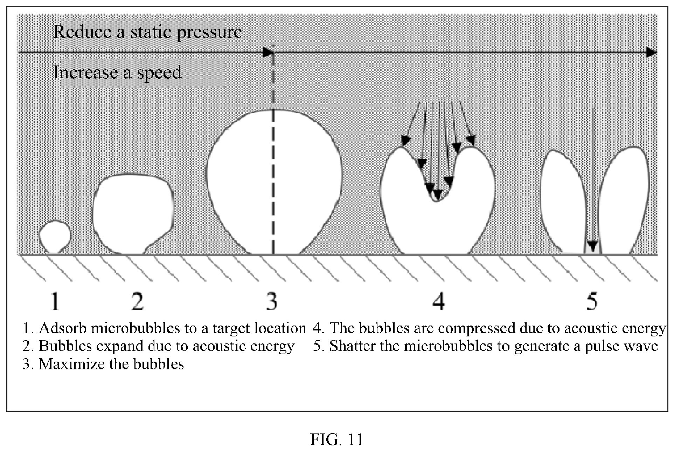

FIG. 11 is a schematic explanatory diagram of a cavitation. The cavitation, also referred to as the cavitation phenomenon, refers to the effect generated by microbubbles, which is induced when ultrasonic waves with certain energy and audio frequency are applied to the microbubbles.

In this embodiment, the cavitation is an inertial cavitation. When the microbubbles are introduced into a blood vessel and adsorbed to a target location, the ultrasonic waves can work. When the microbubbles are extremely compressed and expanded due to strong acoustic energy of the ultrasonic waves, shock waves are generated to shatter the microbubbles, so that the microbubbles finally collapse to generate a pulse wave. The forces can remodel a tissue, fat or calcified structure accumulated at an obstructed part.

To sum up, the ultrasonic detection auxiliary system for a catheter and the method therefor according to the present application feature at least the following: The cavitation is induced by emitting ultrasonic waves from the transmitting probe among a plurality of ultrasonic probes, and an ultrasonic reflection signal is received by the receiving probe to learn about the vascular calcification fragmentation status, so as to adjust an intensity and a direction of ultrasonic waves in time, and effectively alleviate arterial vascular obstruction or refractory vascular calcification.

The foregoing embodiments are only the preferred embodiments provided to fully illustrate the present application, and the protection scope of the present application is not limited thereto. Any equivalent substitution or transformation made by a person skilled in the art based on the present application falls within the protection scope of the present application. The protection scope of the present application is subject to the claims.

## Claims

1. An ultrasonic detection auxiliary system for a catheter, suitable for being introduced into a cardiovascular system to alleviate obstruction in the cardiovascular system or a surrounding tissue thereof, comprising:
an ultrasonic balloon catheter with at least two ultrasonic probes configured to continuously emit an ultrasonic wave and obtain a calcified vascular reflection signal; and
a control host, further comprising:
a signal receiving unit electrically connected to the at least two ultrasonic probes and configured to receive a calcification reflection analog signal;
a digital-to-analog conversion circuit configured to receive the calcified vascular reflection signal and generate a calcification characteristic signal;
a duplex circuit coupled to the signal receiving unit and the digital-to-analog conversion circuit;
a processing unit electrically connected to the digital-to-analog conversion circuit and configured to analyze and calculate the calcification characteristic signal to generate a correlation, a sample entropy, a Shannon entropy, an approximate entropy, and an envelope SNR, generate a calcification data value according to the correlation, and generate a control signal according to the calcification data value, the sample entropy, the Shannon entropy, the approximate entropy, and the envelope SNR to enhance or reduce output energy of the ultrasonic wave; and
an amplification circuit electrically connected to the processing unit and configured to amplify a resonance signal according to the control signal, to cause the ultrasonic probes to emit the ultrasonic wave.

2. The ultrasonic detection auxiliary system for a catheter according to claim 1, wherein the ultrasonic balloon catheter comprises a multi-lumen catheter, a flexible hose with at least one opening, and a balloon made of a polymer material and provided with an expansion part for expansion and contraction and necks arranged at both ends of the expansion part respectively, wherein the balloon is fixedly arranged outside the multi-lumen catheter by using the necks, and the expansion part and the necks form an expansion space outside the multi-lumen catheter, to inject a fluid composition with microbubbles through each of the openings.

3. The ultrasonic detection auxiliary system for a catheter according to claim 2, wherein the at least two ultrasonic probes are arranged inside the expansion part of the balloon, sleeved on the multi-lumen catheter, and configured to emit an ultrasonic wave to induce an inertial cavitation generated by the microbubbles in the fluid composition located in the expansion space.

4. The ultrasonic detection auxiliary system for a catheter according to claim 2, wherein the multi-lumen catheter comprises an inner tube and an outer tube, which are arranged as a concentric circle structure, the inner tube forms a guide wire lumen, a guide wire is arranged in the guide wire lumen, the ultrasonic balloon catheter slides between blood vessels by using the guide wire, the expansion part of the balloon is placed at a vascular calcified part, and the outer tube has at least one lumen, which comprises a microbubble lumen for injecting the fluid composition, and a power lumen for allowing a wire providing power required by the ultrasonic probes to pass through.

5. The ultrasonic detection auxiliary system for a catheter according to claim 4, wherein in the multi-lumen catheter, the inner tube has an outer diameter of 0.5 mm to 1 mm and an inner diameter of 0.3 mm to 0.8 mm, and the outer tube has an outer diameter of 2 mm and an inner diameter of 0.5 mm to 1 mm.

6. The ultrasonic detection auxiliary system for a catheter according to claim 2, wherein the ultrasonic balloon catheter further comprises a catheter assembly, one end of the catheter assembly is connected to the multi-lumen catheter, the other end thereof is provided with a guide wire channel and a fluid composition channel, the guide wire channel allows the guide wire to pass through to enter the guide wire lumen, and the fluid composition channel allows the fluid composition to be injected into the microbubble lumen.

7. The ultrasonic detection auxiliary system for a catheter according to claim 1, wherein the at least two ultrasonic probes comprise a transmitting probe for emitting the ultrasonic wave and a receiving probe for obtaining the calcified vascular reflection signal, and the transmitting probe and the receiving probe are arranged on the ultrasonic balloon catheter at an interval.

8. The ultrasonic detection auxiliary system for a catheter according to claim 1, wherein the at least two ultrasonic probes are each a tubular ultrasonic probe, and the ultrasonic probe has an inner diameter of 0.5 mm to 1 mm, an outer diameter of 1.5 mm to 2.5 mm, a height of 2 mm to 6 mm, and resonant frequencies of 100 kHz to 2 MHz and 2 MHz to 10 MHz.

9. The ultrasonic detection auxiliary system for a catheter according to claim 1, wherein grounds on outer surfaces of the at least two ultrasonic probes are segmented by cutting out gaps of 0.01 mm to 0.2 mm at interval between 90° and 180°.

10. An ultrasonic detection auxiliary method for a catheter, suitable for being introduced into a cardiovascular system to alleviate obstruction in the cardiovascular system or a surrounding tissue thereof, comprising the following steps:
emitting an ultrasonic wave by an ultrasonic probe in an ultrasonic balloon catheter, and obtaining a calcified vascular reflection signal by another ultrasonic probe;
receiving a calcification reflection analog signal by a signal receiving unit of a control host;
converting the calcified vascular reflection signal into a calcification characteristic signal by a digital-to-analog conversion circuit;
analyzing and calculating, by a processing unit, the calcification characteristic signal to generate a correlation, and performing cross correlation comparison between the correlation and a sample in a database to determine a calcification type and generate a calcification data value;
separately calculating, by the processing unit, a sample entropy, a Shannon entropy, an approximate entropy, and an envelope SNR according to the calcification characteristic signal, and performing comparison with a sample entropy threshold, a Shannon entropy threshold, an approximate entropy threshold, and an envelope SNR threshold in the database respectively, so as to determine a calcification fragmentation level;
determining, by the processing unit, whether vascular calcification fragmentation is completed according to the calcification data value and the calcification fragmentation level; and
generating a control signal to stop emitting the ultrasonic wave, when the vascular calcification fragmentation has been completed, or
generating the control signal, when the vascular calcification fragmentation is not completed, adjusting an intensity of the ultrasonic wave by an amplification circuit, and after a preset time, obtaining the calcified vascular reflection signal again by the signal receiving unit and performing analysis and calculation.
